(19) Europäisches Patentamt

European Patent Office

Office européen des brevets

(11) **EP 0 736 325 B1**

(12) **EUROPÄISCHE PATENTSCHRIFT**

(45) Veröffentlichungstag und Bekanntmachung des Hinweises auf die Patenterteilung:
**08.08.2001 Patentblatt 2001/32**

(51) Int Cl.[7]: **B01J 23/56**, C07C 68/00, C07C 69/96

(21) Anmeldenummer: **96104709.9**

(22) Anmeldetag: **25.03.1996**

(54) **Verwendung von Platinmetall enthaltenden Träger-Katalysatoren zur Herstellung von Diarylcarbonaten**

Use of supported platinum metal catalysts for the preparation of diaryle carbonates

Utilisation des catalyseurs supportés comprennant un métal du groupe du platine pour la préparation de carbonates de diaryle

(84) Benannte Vertragsstaaten:
**DE ES FR GB IT NL**

(30) Priorität: **05.04.1995 DE 19512615**

(43) Veröffentlichungstag der Anmeldung:
**09.10.1996 Patentblatt 1996/41**

(73) Patentinhaber: **BAYER AG**
**51368 Leverkusen (DE)**

(72) Erfinder:
• **Buysch, Hans-Josef, Dr.**
 **47809 Krefeld (DE)**

• **Hesse, Carsten, Dr.**
 **47800 Krefeld (DE)**
• **Jentsch, Jörg-Dietrich, Dr.**
 **45468 Mülheim (DE)**
• **Rechner, Johann, Dr.**
 **47906 Kempen (DE)**
• **Zirngiebl, Eberhard, Dr.**
 **51061 Köln (DE)**

(56) Entgegenhaltungen:
**EP-A- 0 464 460          EP-A- 0 581 240**
**EP-A- 0 607 943          EP-A- 0 614 876**
**EP-A- 0 654 461          DE-A- 2 815 512**

**Beschreibung**

Die vorliegende Erfindung betrifft die Verwendung von Platinmetall enthaltende Träger-Katalysatoren zur Herstellung von Diarylcarbonaten durch Umsetzung von aromatischen Hydroxyverbindungen mit Kohlenmonoxid und Sauerstoff, die dadurch gekennzeichnet sind, daß die Träger-Katalysatoren zusätzlich zum Platinmetall noch mindestens einen Cokatalysator enthalten.

Es ist bekannt, organische Carbonate durch oxidative Umsetzung von aromatischen Hydroxyverbindungen mit Kohlenmonoxid in Gegenwart eines Edelmetall-Katalysators herzustellen (DE-OS 28 15 512). Als Edelmetall wird bevorzugt Palladium eingesetzt. Zusätzlich können ein Cokatalysator (z.B. Mangan- oder Kobaltsalze), eine Base, ein quartäres Salz, verschiedene Chinone bzw. Hydrochinone und Trockenmittel eingesetzt werden. Dabei kann in einem Lösungsmittel, bevorzugt in Methylenchlorid, gearbeitet werden.

Für die wirtschaftliche Durchführung dieses Prozesses ist neben der Aktivität und der Selektivität die effektive Wiedergewinnung des Edelmetall-Katalysators von entscheidender Bedeutung: Zum einen stellt der Edelmetall-Katalysator einen erheblichen Kostenfaktor dar. Verluste an Edelmetall-Katalysator müssen kostenintensiv ersetzt werden. Zum anderen dürfen keine Reste des Edelmetall-Katalysators im Produkt verbleiben. Für den Prozeß der oxidativen Carbonylierung von aromatischen Hydroxyverbindungen zu Diarylcarbonaten ist die wirtschaftliche und effiziente Rückgewinnung homogener Katalysatoren bisher nicht beschrieben. Mit geringerem Aufwand kann die Abtrennung eines Edelmetall-Katalysators aus einer flüssigen Reaktionsmischung z.B durch Filtrieren oder Zentrifugieren erfolgen, wenn man heterogene, z.B. Träger-Katalysatoren einsetzt.

Zur Herstellung von Träger-Katalysatoren geeignete Materialien sind bekannt. Je nach Art des Prozesses benutzt man Träger mit großer innerer Oberfläche, wie z.B. Aluminiumoxid, Magnesiumoxid, Aktivkohle oder Siliciumdioxid mit mehr als 50 m$^2$ Oberfläche pro Gramm, Träger mit Oberflächen um 5 m$^2$/g und entsprechend größeren Porenradien, wie z.B. Ruß, Titandioxid, Eisenoxid oder Zinkoxid oder grobkörnige Träger, wie z.B. Siliciumcarbid und Korund (Ullmanns Enzyklopädie der technischen Chemie, 3. Auflage, Berlin/München 1957, Band 9, S. 263 ff). Grundsätzlich können sowohl synthetische Materialien, wie aktivierte Aluminiumoxide, Kieselgele, Silikate, Titandioxide oder Aktivkohlen, als auch solche aus natürlichen Quellen, wie z.B. Bimsstein, Kaolin, Bleicherden, Bauxite, Bentonite, Kieselgur, Asbest oder Zeolithe, verwendet werden. In EP 572 980, EP 503 581 und EP 614 876 werden Edelmetall-Träger-Katalysatoren verwendet, die 5% Pd auf Kohleträgern enthalten. Jedoch liefern derartige Träger-Katalysatoren nach eigenen Untersuchungen nur sehr unbefriedigende oder gar keine Umsätze, so daß auch diese für eine wirtschaftliche Prozeßführung nicht geeignet sind. In JP 01/165 551 (zitiert nach C.A. 112 (1990), 76618j) wird beschrieben, daß für die Herstellung von aromatischen Carbonaten Palladium oder Pd-Verbindungen, wie Pd-acetylacetonat, in Kombination mit (Erd)Alkalijodiden oder Oniumjodiden, wie Tetrabutylammoniumjodid, und mindestens einem Zeolith eingesetzt werden können. In JP 04/257 546 und JP 04/261 142 wird in je einem Beispiel ein Träger-Katalysator zur Herstellung von aromatischen Carbonaten beschrieben, bei dem Siliciumcarbid-Granulat als Trägermaterial für einen Träger-Katalysator in einer Destillationskolonne verwendet wird. Obwohl in den betreffenden Beispielen unter drastischen Bedingungen (hoher Druck, hohe Temperatur) gearbeitet wird, ermöglicht dieser Katalysator nur sehr geringe Raum-Zeit-Ausbeuten. Diese niedrigen Raum-Zeit-Ausbeuten machen eine ökonomische Herstellung von aromatischen Carbonaten mit derartigen Träger-Katalysatoren unmöglich.

Es steht also bisher kein Träger-Katalysator zur Verfügung, mit dem Diarylcarbonate durch Umsetzung einer aromatischen Hydroxyverbindung mit Kohlenmonoxid und Sauerstoff ökonomisch effizient hergestellt werden können. Es bestand daher die Aufgabe, einen Träger-Katalysator mit hoher Aktivität und Selektivität zu finden, der die ökonomisch effiziente Herstellung von Diarylcarbonaten durch Umsetzung einer aromatischen Hydroxyverbindung mit Kohlenmonoxid und Sauerstoff erlaubt.

Es wurde nun gefunden, daß die dargestellten Nachteile überwunden werden können, wenn Platinmetall-Trägerkatalysatoren eingesetzt werden, die zusätzlich zum Platinmetall noch mindestens einen Co-Katalysator enthalten. Die Katalysatoren aus Platinmetall und mindestens einem Co-Katalysator auf einem Träger werden erfindungsgemäß als Pulver, Tabletten oder bindemittelhaltige Extrudate eingesetzt. Geeignete Bindemittel sind z.B. SiO$_2$, Al$_2$O$_3$ oder Tonmineralien. Die Bindemittelgehalte können in einem breiten Bereich, beispielsweise von 0,5 bis 99,5 Gew.-%, bezogen auf das Gesamtgewicht des Trägers, variiert werden.

Die erfindungsgemäß verwendeten Träger-Katalysatoren enthalten im reaktionsbereiten Zustand (i) ein Platinmetall, ein Platinmetall-Halogenid oder eine Platinmetall-Halogenid enthaltende Komplexverbindung oder eine Verbindung, die unter Reaktionsbedingungen in ein Platinmetall, ein Platinmetall-Halogenid oder eine Platinmetall-Halogenid enthaltende Komplexverbindung übergeführt werden kann, in einer Menge von 0,01 - 15 Gew.-%, bevorzugt 0,05 - 10 Gew.-%, gerechnet als Platinmetall und bezogen auf das Gesamtgewicht des Katalysators, und (ii) eine cokatalytisch wirkende Metallverbindung eines Elementes der Gruppe Mn, Cu, Co, V, Mo in einer Menge von 0,01 - 15 Gew.-%, bevorzugt 0,05 - 10 Gew.-%, gerechnet als Metall und bezogen auf das Gesamtgewicht des Katalysators.

Solche Katalysatoren liegen als heterogen katalytische Systeme vor und erleichtern somit die Abtrennung des Reaktionsproduktes vom teuren Platinmetall, dessen Verbindungen und dem Cokatalysator.

Die Erfindung betrifft die Verwendung der oben genannten Katalysatoren in einem Verfahren zur Herstellung eines aromatischen Carbonats der Formel

$$R\text{-}O\text{-}CO\text{-}O\text{-}R \qquad (I),$$

in der

R    substituiertes oder nicht substituiertes $C_6$-$C_{12}$-Aryl, bevorzugt substituiertes oder nicht substituiertes Phenyl, besonders bevorzugt nicht substituiertes Phenyl bedeutet,

durch Umsetzung einer aromatischen Hydroxyverbindung der Formel

$$R\text{-}O\text{-}H \qquad (II),$$

in der R die oben angegebene Bedeutung hat,
mit Kohlenmonoxid und Sauerstoff in Gegenwart eines quartären Ammonium- oder Phosphonium-Salzes und einer Base bei 30 bis 200°C, bevorzugt 30-150°C, besonders bevorzugt 40 bis 120°C und bei einem Druck von 1 bis 150 bar, bevorzugt 2-50 bar, besonders bevorzugt 5 bis 25 bar.

Am Beispiel der Bildung von Diphenylcarbonat kann das Verfahren wie folgt formelmäßig dargestellt werden:

$$2\ C_6H_5\text{-}OH + CO + \tfrac{1}{2}\ O_2 \rightarrow (C_6H_5O)_2CO + H_2O$$

Für die erfindungsgemäße Verwendung eignen sich als Katalysatorträger alle technisch üblichen Katalysatorträger, z.B. solche auf der Basis von Kohle, Elementoxiden, Elementcarbiden oder Elementsalzen in verschiedenen Anwendungsformen. Beispiele für kohlenstoffhaltige Träger sind Koks, Graphit, Ruß oder Aktivkohle. Beispiele für Elementoxid-Katalysatorträger sind $SiO_2$ (natürliche oder synthetische Kieselsäuren, Quarz), $Al_2O_3$ ($\alpha$-, $\gamma$-$Al_2O_3$), Tonerden, natürliche und synthetische Alumosilicate (Zeolithe), $TiO_2$ (Rutil, Anatas), $ZrO_2$ oder ZnO. Beispiele für Elementcarbide und -salze sind SiC, $AlPO_4$, $BaSO_4$, $CaCO_3$ u.a. Sie können sowohl im Sinne chemisch einheitlicher Reinsubstanzen als auch im Gemisch eingesetzt werden. Für die erfindungsgemäße Verwendung als Katalysatorträger eignen sich sowohl stückige als auch pulverförmige Materialien. Für den Fall der Anordnung des Träger-Katalysators als Festbett wird der Träger vorzugsweise als Formkörper, z.B. als Kugeln, Zylinder, Stäbchen, Hohlzylinder, Ringe, usw. eingesetzt. Wahlweise können Katalysatorträger weiter durch Extrudieren, Tablettieren, gegebenenfalls unter Zumischen weiterer Katalysatorträger oder Bindemittel wie $SiO_2$ oder $Al_2O_3$, und Calcinieren modifiziert werden. Darstellung und Weiterverarbeitung der erfindungsgemäß verwendeten Katalysatorträger sind dem Fachmann wohl bekannt und Stand der Technik.

Die Reaktivkomponente des verwendeten Katalysators besteht im reaktionsbereiten Zustand aus einem Platinmetall, einem Platinmetall-Halogenid, wie $PdCl_2$ oder $PdBr_2$, oder einer Platinmetall-Halogenid enthaltenden Komplexverbindung, die außerdem beispielsweise Olefine, Amine, Phosphine, Nitrile, Kohlenmonoxid oder Wasser enthalten kann, wie $A_2(PdHal_4)$, wobei A beispielsweise für Li, Na, K, $NH_4$, Rb, Cs, $NR^1_4$ mit $R^1$: organischer Rest $C_6$- bis $C_{10}$-Aryl-, $C_7$- bis $C_{12}$-Aralkyl- und/oder $C_1$- bis $C_{20}$-Alkyl-Rest, und Hal für ein Halogen, wie beispielsweise F, Cl, Br, I steht sowie mindestens einem Cokatalysator. Geeignete Platinmetall-Komplexverbindungen sind grundsätzlich bekannt. Beispiele sind: $Li_2(PdCl_4)$, $Na_2(PdCl_4)$, $K_2(PdCl_4)$, $(NBu)_2$$(PdCl_4)$, $Na_2(PdBr_4)$, $K_2(PdBr_4)$, $(NBu_4)_2(PdBr_4)$ (mit Bu = n-Butyl), Beispiele für olefinhaltige Platinmetallkomplexe sind [Allylpalladiumchlorid]-Dimer -$[C_3H_5PdCl]_2$, 1,5-Cyclooctadienpalladiumdichlorid -$C_8H_5PdCl_2$, Beispiele für phosphinhaltige Platinmetallkomplexe sind [1,2-Bis(diphenylphosphino)ethan]palladiumdichlorid - $Pd[(C_6H_5)_2PCH_2CH_2P(C_6H_5)_2]Cl_2$, Bis(triphenylphosphino)palladiumdichlorid -$Pd[P(C_6H_5)_3]_2Cl_2$, Beispiele für aminhaltige Platinmetallkomplexe sind Diaminopalladiumdibromid -$Pd(NH_3)_2Br_2$, Diaminopalladiumdichlorid -$Pd(NH_3)_2Cl_2$, Tetraaminopalladiumtetrachloropalladat -$[Pd(NH_3)_4][PdCl_4]$, Beispiele für nitrilhaltige Platinmetallkomplexe sind Bis(acetonitril)palladiumdichlorid -$Pd(CH_3CN)_2Cl_2$, Bis(benzonitril)palladiumdichlorid, -$Pd(C_6H_5CN)_2Cl_2$, Beispiele für kohlenmonoxidhaltige Platinmetallkomplexe sind Tetrabutylammoniumtribromocarbonylpalladat -$(NBu_4)Pd(CO)Br_3$ (mit Bu = n-Butyl) und Tetrabutylammoniumtrichlorocarbonylpalladat -$(NBu_4)Pd(CO)Cl_3$ (mit Bu = n-Butyl). In den genannten Beispielen wurde Pd als Platinmetall genannt, jedoch kommen auch andere Platinmetalle in Betracht, wie Pt, Ir, Ru oder Rh; Pd und Rh sind jedoch bevorzugt, insbesondere Pd. Das Platinmetall liegt in einer Wertigkeitsstufe von 0 bis 4 vor.

Es hat sich weiterhin gezeigt, daß das Platinmetall-Halogenid bzw. die das Platinmetall-Halogenid enthaltende Komplexverbindung in situ während der Präparation oder während des Katalysatoreinsatzes unter Reaktionsbedingungen aus einer geeigneten halogenfreien Platinmetall-Verbindung und einer Halogenid enthaltenden Verbindung auf dem Träger hergestellt werden kann. Als halogenfreie Platinmetall-Verbindungen kommen z.B. Platinmetallnitrate, -acetate, -propionate, -butyrate, -oxalate, -carbonate, -oxide, -hydroxide, -acetylacetonate und andere dem Fachmann geläufige in Frage. Als Halogenid enthaltende Verbindungen kommen halogenhaltige Salze und Komplexverbindungen der Elemente der ersten bis fünften Hauptgruppe und der ersten bis achten Nebengruppe des Periodensystems der Elemente (Mendelejew) sowie der Seltenerdmetalle (Atomnummern 58-71) oder aliphatische Halogenkohlenwasserstoffe in Frage. Beispiele sind NaBr, NaCl, MgCl$_2$, MgBr$_2$, AlCl$_3$, CH$_2$Cl$_2$, NaPF$_6$, MnCl$_2$, MnBr$_2$, CoBr$_2$, CeCl$_3$, SmI$_2$, CuCl$_2$, Na$_2$ZnCl$_4$, TiCl$_4$ und NR$^1_4$Br, wobei R$^1$ die oben genannte Bedeutung hat.

Die Menge des Platinmetalls, Platinmetall-Halogenids oder der das Platinmetall-Halogenid enthaltenden Komplexverbindung im reaktionsbereiten Zustand beträgt 0,01 bis 15 Gew.-%, bevorzugt 0,05 bis 10 Gew.-%, gerechnet als Platinmetall und bezogen auf das Gesamtgewicht des Katalysators.

Als Cokatalysator für die erfindungsgemäße Verwendung wird eine Metallverbindung eines Elementes der Gruppe Mn, Cu, Co, V, und Mo, besonders bevorzugt Mn, Co, Cu, Mo. Die Metalle können beispielsweise als Halogenide, Oxide, Carboxylate von C$_2$-C$_6$-Carbonsäuren, Diketonate oder Nitrate sowie als Komplexverbindungen, die beispielsweise Kohlenmonoxid, Olefine, Amine, Nitrile, Phosphine und Halogenide enthalten können, eingesetzt werden.

Die Menge der Cokatalysator enthaltenden Verbindung im reaktionsbereiten Zustand beträgt 0,01 bis 15 Gew.-%, bevorzugt 0,05 bis 10 Gew.-%, gerechnet als Metall und bezogen auf das Gesamtgewicht des Katalysators.

Platinmetall und Cokatalysator können gleichzeitig, d.h. aus einer gemeinsamer Lösung, oder nacheinander in beliebiger Reihenfolge auf den Träger aufgebrach werden. Geeignete Lösungsmittel für die Platinmetall- und Cokatalysator-Verbindungen zur Herstellung von erfindungsgemäßen Träger-Katalysatoren sind z.B Wasser, aliphatische Kohlenwasserstoffe, wie Pentan, n-Hexan, Cyclohexan usw aliphatische halogenierte Kohlenwasserstoffe, wie Dichlormethan, Trichlormethan usw., ungesättigte Kohlenwasserstoffe, wie Penten, Isopren, Cyclopentadien ; Hexene, Hexine, Cyclohexene, Cyclooctadiene usw., aromatische Kohlenwasserstoffe, wie Benzol, Toluol, Xylol usw., halogenierte aromatische Kohlenwasserstoffe, wie Chlorbenzol, Dichlorbenzol, usw., primäre, sekundäre oder tertiäre Alkohole, wie Methanol, Ethanol, n-Propanol, iso-Propanol, n-Butanol, iso-Butanol, t-Butanol, Cumylalkohol, iso-Amylalkohol, Diethylenglykol, usw., Ketone, wie Aceton, 2-Butanon, Methylisobutylketon, Acetylaceton usw., Ether, wie Diethylether, Diisopropylether, Methylt-butylether, Dioxan, Tetrahydrofuran, usw., Ester, wie Methylacetat, Ethylacetat, usw., Nitrile, wie Acetonitril, Benzonitril, usw., Carbonate, wie Dimethylcarbonat, Diethylcarbonat, Diphenylcarbonat, usw., Amide, wie Dimethylacetamid, N-Methylpyrrolidinon und Tetramethylharnstoff genannt. Selbstverständlich können auch Mischungen solcher Lösungsmittel eingesetzt werden.

Die Herstellung der erfindungsgemäß verwendeten Katalysatoren erfolgt nach Methoden, die dem Fachmann grundsätzlich bekannt sind. So können Lösungen eines oder mehrerer der genannten Platinmetall-Verbindungen und der genannten halogenidhaltigen Verbindungen sowie eines oder mehrerer Cokatalysatoren beispielsweise durch Tränken, Adsorption, Tauchen, Sprühen, Imprägnieren und Ionenaustausch auf den erfindungsgemäß einzusetzenden Katalysatorträger gebracht werden. Es ist weiterhin möglich, ein oder mehrere Platinmetalle, die genannten halogenidhaltigen Verbindungen sowie einen oder mehrere Cokatalysatoren durch Fällung mit einer Base auf dem Träger zu fixieren. Als Base kommen z.B. (Erd-)Alkalimetallhydroxide, wie Ca(OH)$_2$, Mg(OH)$_2$, NaOH, LiOH und KOH, (Erd-)Alkalimetallhydrogencarbonate wie Ca(HCO$_3$)$_2$, Mg(HCO$_3$)$_2$, NaHCO$_3$, LiHCO$_3$ und KHCO$_3$, (Erd-)Alkalimetallcarbonate wie CaCO$_3$, MgCO$_3$, Na$_2$CO$_3$, Li$_2$CO$_3$ und K$_2$CO$_3$, Alkalimetallsalze schwacher organischer Säuren, wie Natriumacetat, Kaliumacetat und Lithiumacetat, und (Erd-)Alkalimetallsalze von substituierten oder nicht substituierten Phenolen (bei substituierten Phenolen handelt es sich um solche, wie sie weiter unten als in das Verfahren zur Diarylcarbonat-Herstellung einsetzbare beschrieben werden), wie Lithiumphenolat, Natriumphenolat, -Natriumkresolat und Kaliumphenolat in Frage. Das Platinmetall und die halogenidhaltige Verbindung können sowohl in beliebiger Reihenfolge nacheinander als auch gleichzeitig auf den Träger gebracht werden. Eine spezielle Ausführungsform der Erfindung beinhaltet das Aufbringen des Platinmetalls durch Fällung eines Platinmetallhalogenids oder einer Platinmetallhalogenid-Komplexverbindung mit einer geeigneten Base (in Frage kommen z.B. solche, wie sie oben beschrieben sind), Reduktion der gefällten Platinmetall-Base zum Metall mit einem geeigneten Reduktionsmittel, wie beispielsweise Hydrazin, Formaldehyd, Na-Formiat, NaBH$_4$ bei Temperaturen zwischen 0°C und 200°C oder gasförmigem Wasserstoff bei Temperaturen zwischen 0°C und 500°, bevorzugt zwischen 20 und 300°C, besonders bevorzugt 30-250°C und Umsetzung des Platinmetalls mit Halogenwasserstoff oder gasförmigem Halogen bei Temperaturen zwischen 20°C und 600°C, bevorzugt 50 und 500°C.

Während der Aufbringung von Platinmetall und Cokatalysator auf den Träger kann die Mischung gerührt werden. Es kann aber auch vorteilhaft sein, die Mischung stehenzulassen oder zu schütteln, damit gegebenenfalls verwendete Formkörper nicht durch einen Rührer beschädigt werden. Nach Aufbringung von Platinmetall und Cokatalysator auf den Träger wird der Träger-Katalysator z.B. durch Filtrieren, Sedimentieren oder Zentrifugieren abgetrennt. In einer weiteren Ausführungsform der Erfindung wird das Lösungsmittel durch Abdestillieren abgetrennt.

Nach Abtrennung des Lösemittels werden die so erhaltenen Träger-Katalysatoren getrocknet. Dies kann an der Luft, im Vakuum oder im Gasstrom geschehen. Geeignete Gase für die Trocknung des Träger-Katalysators im Gasstrom sind Stickstoff, Sauerstoff, Kohlenmonoxid, Kohlendioxid oder Edelgase sowie beliebige Mischungen der genannten Gase, bevorzugt z.B.

Luft. Ebenfalls eignen sich gasförmige Kohlenwasserstoffe, wie Alkane (z.B. Methan, Ethan, Propan), Alkene, wie Ethen, Propen, Butene, Butadien und Alkine, wie Ethin, Propin usw. in beliebiger Zusammensetzung. Die Trocknung erfolgt bei 20 bis 200°C, bevorzugt bei 40 bis 180°C, besonders bevorzugt bei 60 bis 150°C. Die Trockenzeit hängt z.B. von der Porosität des verwendeten Träger und vom verwendeten Lösungsmittel ab. Sie beträgt im allgemeinen einige Stunden, beispielsweise 0,5 bis 50 h, bevorzugt 1 bis 40 h, besonders bevorzugt 1 bis 30 h.

Nach der Trocknung können die getrockneten Träger-Katalysatoren calciniert werden. Dies kann an der Luft, im Vakuum oder im Gasstrom geschehen. Geeignete Gase für die Calcinierung des Träger-Katalysators im Gasstrom sind z.B. Stickstoff, Sauerstoff, Kohlendioxid oder Edelgase sowie beliebige Mischungen der genannten Gase, bevorzugt z.B. Luft. Die Calcinierung erfolgt bei 100 bis 800°C, bevorzugt bei 100 bis 700°C, besonders bevorzugt bei 100 bis 600°C. Hierbei kann es ggf. von Vorteil sein, wenn während der Calcinierung die Zusammensetzung des Gases sprunghaft oder kontinuierlich geändert wird. Eine sprunghafte Änderung der Calciniergaszusammensetzung kann beispielsweise erfolgen, indem man nach 10 h den $O_2$-Gehalt von 10 Vol.-% auf 20 Vol.-% für die restlichen 10 h erhöht und die Temperatur beibehält. Eine kontinuierliche Änderung der Calciniergaszusammensetzung kann beispielsweise erfolgen, indem man unter Beibehaltung der Temperatur innerhalb von 20 h den Sauerstoffgehalt von 0 Vol.-% auf 20 Vol.-% mit 1 Vol.-% Steigerung/h erhöht. Die Calcinierungszeit beträgt im allgemeinen einige Stunden, beispielsweise 0,5 bis 50 h, bevorzugt 1 bis 40 h, besonders bevorzugt 1 bis 30 h.

Bei den mit den erfindungsgemäß verwendeten Träger-Katalysatoren umsetzbaren aromatischen Hydroxyverbindungen handelt es sich beispielsweise um Phenol, o-, moder p-Kresol, o-, m- oder p-Chlorphenol, o-, m- oder p-Ethylphenol, o-, m- oder p-Propylphenol, o-, m- oder p-Methoxyphenol, 2,6-Dimethylphenol, 2,4-Dimethylphenol, 3,4-Dimethylphenol, 1-Naphthol, 2-Naphthol und Bisphenol A, bevorzugt um Phenol. Allgemein handelt es sich im Falle einer Substitution der aromatischen Hydroxyverbindung um 1 oder 2 Substituenten in der Bedeutung von $C_1$-$C_4$-Alkyl, $C_1$-$C_4$-Alkoxy, Fluor, Chlor oder Brom.

Bei der erfindungsgemäßen Verwendung des Katalysators können beliebige sowohl organische als auch anorganische Basen oder Mischungen derselben eingesetzt werden. Als Beispiele für anorganische Basen seien, ohne das Verfahren einzuschränken, Alkalimetallhydroxide und -carbonate, -carboxylate oder andere Salze schwacher Säuren sowie Alkalisalze von aromatischen Hydroxyverbindungen der Formel (II), z. B. Alkalimetallphenolate, genannt. Selbstverständlich können in das erfindungsgemäße Verfahren auch die Hydrate von Alkalimetallphenolaten eingesetzt werden. Als Beispiel für ein solches Hydrat sei hier, ohne das Verfahren einzuschränken, Natriumphenolat-Trihydrat genannt. Die Menge an zugesetztem Wasser ist jedoch vorzugsweise so bemessen, daß pro Mol Base maximal 5 Mol Wasser eingesetzt werden. Höhere Wasserkonzentrationen führen i.a. zu schlechteren Umsätzen und Zersetzung gebildeter Carbonate. Als organische Basen seien, ohne das Verfahren einzuschränken, tertiäre Amine, die als organische Reste $C_6$- bis $C_{10}$-Aryl-, $C_7$- bis $C_{12}$-Aralkyl- und/oder $C_1$- bis $C_{20}$-Alkyl-Reste tragen können oder Pyridinbasen oder hydrierte Pyridinbasen darstellen, genannt, beispielsweise Triethylamin, Tripropylamin, Tributylamin, Trioctylamin, Benzyldimethylamin, Dioctylbenzylamin, Dimethyl-phenethylamin, 1-Dimethylamino-2-phenylpropan, Pyridin, N-Methylpiperidin, 1,2,2,6,6-Pentamethylpiperidin. Bevorzugt wird als Base ein Alkalisalz einer aromatischen Hydroxyverbindung verwendet, besonders bevorzugt ein Alkalisalz der aromatischen Hydroxyverbindung (II), die auch zum organischen Carbonat umgesetzt werden soll. Diese Alkalisalze können Lithium-, Natrium-, Kalium-, Rubidium- oder Cäsiumsalze sein. Bevorzugt werden Lithium-, Natrium-, und Kaliumphenolat, besonders bevorzugt Natriumphenolat eingesetzt.

Die Base kann dem Reaktionsgemisch als reine Verbindung in fester Form oder als Schmelze zugesetzt werden. In einer weiteren Ausführungsform der Erfindung wird die Base dem Reaktionsgemisch als Lösung, die 0,1 bis 80 Gew.-%, bevorzugt 0,5 bis 65 Gew.-%, besonders bevorzugt 1 bis 50 Gew.-% der Base enthält, zugesetzt. Als Lösungsmittel können hierbei sowohl Alkohole oder Phenole, wie z.B. das umzusetzende Phenol (II), als auch inerte Lösungsmittel verwendet werden. Als solche seien die weiter unten als Reaktionmedien erwähnten genannt.

Diese Lösungsmittel können allein oder in beliebiger Kombination miteinander eingesetzt werden. So besteht eine Ausführungsform des Verfahrens beispielsweise darin, daß man die Base in einer Phenolschmelze löst, die mit einem Lösungsmittel verdünnt wurde. Bevorzugt wird die Base in der Schmelze einer aromatischen Hydroxyverbindung gelöst, besonders bevorzugt in einer Schmelze der aromatischen Hydroxyverbindung (II), die zum organischen Carbonat umgesetzt werden soll. Ganz besonders bevorzugt wird die Base, in Phenol gelöst, zugesetzt. Die Base wird in einer von der Stöchiometrie unabhängigen Menge zugesetzt. Das Verhältnis von Platinmetall, z.B. Palladium, zu Base wird vorzugsweise so gewählt, daß pro Mol Platinmetall, z.B. Palladium, 0,1 bis 500, bevorzugt 0,3 bis 200 besonders bevorzugt 0,9 bis 130 Äquivalente Base, eingesetzt werden.

Das Verfahren wird vorzugsweise ohne Lösungsmittel durchgeführt. Selbstverständlich können auch inerte Lösungsmittel verwendet werden. Als Beispiele für Lösungsmittel seien Dimethylacetamid, N-Methylpyrrolidinon, Dioxan, t-Butanol, Cumylalkohol, Isoamylalkohol, Tetramethylharnstoff, Diethylenglykol, halogenierte Kohlenwasserstoffe (z.B. Chlorbenzol oder Dichlorbenzol) und Ether genannt.

Bei den im Rahmen der vorliegenden Erfindung eingesetzten quartären Salzen kann es sich beispielsweise um mit organischen Resten substituierte Ammonium- oder Phosphoniumsalze handeln. Geeignet für den Einsatz im erfindungsgemäßen Verfahren sind Ammonium- und Phosphoniumsalze, die als organische Reste $C_6$- bis $C_{10}$-Aryl-, $C_7$- bis $C_{12}$-Aralkyl- und/ oder $C_1$- bis $C_{20}$-Alkyl-Reste und als Anion ein Halogenid, Tetrafluoroborat oder Hexafluorophosphat tragen. Bevorzugt werden in das erfindungsgemäße Verfahren Ammoniumsalze, die als organische Reste $C_6$- bis $C_{10}$-Aryl-, $C_7$- bis $C_{12}$-Aralkyl- und/oder $C_1$- bis $C_{20}$-Alkyl-Reste und als Anion ein Halogenid tragen, eingesetzt, besonders bevorzugt ist Tetrabutylammoniumbromid. Die Menge eines solchen quartären Salzes beträgt 0,1 bis 50 Gew.-%, bezogen auf das Gewicht des Reaktionsgemisches. Vorzugsweise beträgt diese Menge 0,5 bis 15 Gew.-%, besonders bevorzugt 1 bis 5 Gew.-%.

Das Verfahren wird, vorzugsweise ohne Lösungsmittel, bei 30 bis 200°C, bevorzugt bei 30 bis 150°C, besonders bevorzugt bei 40 bis 120°C und bei einem Druck von 1 bis 150 bar, bevorzugt von 2 bis 50 bar, besonders bevorzugt bei 5 bis 25 bar durchgeführt.

Die Träger-Katalysatoren können als Pulver oder Formkörper eingesetzt werden und aus der Reaktionsmischung z.B. durch Filtration, Sedimentation oder Zentrifugieren wieder abgetrennt werden.

Die Herstellung von aromatischen Carbonaten unter erfindungsgemäßer Verwendung von Träger-Katalysatoren kann in verschiedenen Ausführungsvarianten ausgeübt werden. Eine Möglichkeit besteht in der diskontinuierlichen Durchführung. Im Falle einer kontinuierlichen Fahrweise im Gegen- oder Gleichstrom oder in der Rieselphase am Festbett-Katalysator werden Belastungen von 0,01 bis 20 g aromatische Hydroxyverbindung pro Gramm Träger-Katalysator und Stunde, bevorzugt 0,05 bis 10 g aromatische Hydroxyverbindung pro Gramm Träger-Katalysator und Stunde, besonders bevorzugt 0,1 bis 5 g aromatische Hydroxyverbindung pro Gramm Träger-Katalysator und Stunde eingestellt. Die in diskontinuierlichen Versuchen verwendeten Träger-Katalysatoren können bei gleichen Einsatzstoffen ohne Reinigung wiederholt eingesetzt werden. Bei kontinuierlicher Arbeitsweise können die eingesetzten Träger-Katalysatoren über lange Zeit im Reaktor verbleiben. Bevorzugt kommt bei der Verwendung erfindungsgemäßer Träger-Katalysatoren eine kontinuierliche Arbeitsweise im Einzelreaktor oder in einer Kaskade aus Reaktoren zum Einsatz.

Wird der Träger-Katalysator als Pulver eingesetzt, sind zur Vermischung der Reaktionskomponenten die zu verwendenden Rührbehälter mit dafür brauchbaren Rührern ausgestattet. Beim Arbeiten mit Träger-Katalysator-Pulvern als Suspension in Rührgefäßen oder Blasensäulen werden Mengen von 0,001 bis 50 Gew.-%, bevorzugt von 0,01 bis 20 Gew.-%, besonders bevorzugt von 0,1 bis 10 Gew.-% Träger-Katalysator-Pulver, bezogen auf die eingesetzte Menge an aromatischer Hydroxyverbindung, verwendet. In besonders bevorzugten Ausführungsformen wird der heterogene Träger-Katalysator als Formkörper ortsfest in Rührkesseln, Blasensäulen, Rieselphasenreaktoren oder Kaskaden dieser Reaktoren, wobei die verschiedenen Reaktortypen auch gleichzeitig in einer Kaskade vorkommen können, eingesetzt.

## Beispiele

### Beispiel 1

a) Belegung eines pulverförmigen Titandioxids mit Palladium und Mangan:

Zu einer Aufschlämmung von 283,5 g Titandioxid-Pulver (Fa. Norton) in 1500 ml Wasser wurden bei Raumtemperatur 300 ml Lösung von 40,5 g (0,16 mol) Mangan(II)-nitrat-4-hydrat in Wasser gegeben. Dann wurde mit verdünnter Natronlauge alkalisch gestellt. Die resultierende Suspension wurde abgesaugt, mit Wasser gewaschen, bei 100°C getrocknet und 3h bei 300°C getempert. Der mit Mangan dotierte Träger wurde in 1500 ml Wasser aufgeschlämmt und mit 300 ml Lösung, enthaltend 50 g Natriumtetrachloropalladat(II)-Lsg. mit 15% Palladium, versetzt. Dann wurde mit verdünnter Natronlauge alkalisch gestellt. Die nunmehr erhaltene Suspension wurde abgesaugt, gewaschen und bei 100°C getrocknet. Der Katalysator enthielt 2,5 Gew.-% Pd und 3 Gew.-% Mn, jeweils als Metall gerechnet.

b) Einsatz des Träger-Katalysators zur Herstellung von Diphenylcarbonat:

In einem Autoklaven (1 l) mit Begasungsrührer, Kühler und nachgeschalteter Kühlfalle wurden bei 80°C, 8,31 g Tetrabutylammoniumbromid in 450 g Phenol gelöst. Dann wurden 4 g des oben beschriebenen Träger-Katalysators und 2,21 g Natriumphenolat, gelöst in 50 g Phenol, zugegeben. Unter Einleitung eines Gasgemisches aus Kohlenmonoxid und Sauerstoff (95:5 Vol.-%) wurde dann der Druck auf 10 bar eingestellt. Die Menge an Gasgemisch wurde auf 300 Nl/h eingestellt. Dem Reaktionsgemisch wurde jede Stunde eine Probe entnommen und gaschromatographisch analysiert. Die Analysen ergaben, daß nach einer Stunde 8,1 % Diphenylcarbonat, nach 2 Stunden 14,3 % Diphenylcarbonat und nach 3 Stunden 18,6 % Diphenylcarbonat im Reaktionsgemisch enthalten waren. In der Kühlfalle waren 14,1 g eines Phenol/Wasser-Gemisches kondensiert.

c) Eine weitere Katalysatorprobe aus Beispiel 1a) wurde unter gleichen Reaktionsbedingungen eingesetzt, mit dem Unterschied, daß zusätzlich ein homogen gelöster Cokatalysator, nämlich 0,77 g Manganacetylacetonat, anwesend war. Dem Reaktionsgemisch wurde jede Stunde eine Probe entnommen und gaschromatographisch analysiert. Die Analysen ergaben, daß nach einer Stunde 8,3 % Diphenylcarbonat, nach 2 Stunden 14,5 % Diphenylcarbonat und nach 3 Stunden 18,5 % Diphenylcarbonat im Reaktionsgemisch enthalten waren. In der Kühlfalle waren 13,9 g eines Phenol/Wasser-Gemisches kondensiert. Die Anwesenheit eines homogen gelösten Cokatalysators ergibt kein besseres Ergebnis und ist somit erfindungsgemäß nicht mehr notwendig.

**Beispiel 2**

a) Belegung eines pulverförmigen Titandioxids mit Palladium und Kobalt:

Zu einer Lösung von 18,75 g Palladium(II)-bromid (0,07 mol), 28,5 g Natriumbromid (0,28 mol) und 33,4 g Kobalt(II)-bromid (0,15 mol) in 1500 ml Wasser wurden bei Raumtemperatur 283,5 g Titandioxid-Pulver (Fa. Norton) gegeben. Dann wurde mit verdünnter Natronlauge alkalisch gestellt. Die Suspension wurde abgesaugt, gewaschen und bei 100°C getrocknet. Der Katalysator enthielt 2,5 Gew.-% Pd und 3 Gew.-% Co, jeweils als Metall gerechnet.

b) Einsatz des Träger-Katalysators zur Herstellung von Diphenylcarbonat:

Der Einsatz des Träger-Katalysators zur Herstellung von Diphenylcarbonat erfolgte wie in Beispiel 1. Die Analysen ergaben, daß nach einer Stunde 6,1 % Diphenylcarbonat, nach 2 Stunden 11,3 % Diphenylcarbonat und nach 3 Stunden 15,0 % Diphenylcarbonat im Reaktionsgemisch enthalten waren. In der Kühlfalle waren 11,5 g eines Phenol/Wasser-Gemisches kondensiert.

**Beispiel 3**

a) Belegung eines Titandioxid-Extrudats mit Palladium und Mangan:

200 ml Titandioxid-Extrudat wurden mit 58,4 ml Lösung von 21,6 g Mangan(II)-chlorid in Wasser getränkt. Dann wurde unter Stickstoff bei 110°C getrocknet. Der mit Mangan dotierte Träger wurde mit 58 ml wäßriger Lösung, enthaltend 33,3 g Natriumtetrachloropalladat(II)-Lsg. mit 15% Palladium, getränkt. Dann wurde unter Stickstoff bei 110°C getrocknet. Der fertige Kontakt enthielt pro Liter 25 g Pd und 30 g Mn, jeweils als Metall gerechnet.

b) Einsatz des Träger-Katalysators zur Herstellung von Diphenylcarbonat:

Der Einsatz des Träger-Katalysators zur Herstellung von Diphenylcarbonat erfolgte wie in Beispiel 1, jedoch mit dem Unterschied, daß sich der Katalysator ortsfest in einem Maschendrahtkörbchen befand. Die Analysen ergaben, daß nach einer Stunde 4,6 % Diphenylcarbonat, nach 2 Stunden 8,7 % Diphenylcarbonat und nach 3 Stunden 11,6 % Diphenylcarbonat im Reaktionsgemisch enthalten waren. In der Kühlfalle waren 9,2 g eines Phenol/Wasser-Gemisches kondensiert.

**Beispiel 4**

a) Belegung eines Titandioxid-Extrudats mit Rhodium und Mangan:

200 ml Titandioxid-Extrudat wurden mit 58,4 ml Lösung von 21,6 g Mangan(II)-chlorid in Wasser getränkt. Dann wurde unter Stickstoff bei 110°C getrocknet. Der mit Mangan dotierte Träger wurde mit 58 ml wäßriger Lösung, enthaltend 12,94 g Rhodium(III)-chloridhydrat, getränkt. Dann wurde unter Stickstoff bei 110°C getrocknet. Der Kontakt enthielt pro Liter 25 g Rhodium und 30 g Mn, jeweils als Metall gerechnet.

b) Einsatz des Träger-Katalysators zur Herstellung von Diphenylcarbonat:

Der Einsatz des Träger-Katalysators zur Herstellung von Diphenylcarbonat erfolgte wie in Beispiel 3. Die Analysen ergaben, daß nach einer Stunde 1,4 % Diphenylcarbonat, nach 2 Stunden 2,9 % Diphenylcarbonat und nach 3 Stunden 4,1 % Diphenylcarbonat im Reaktionsgemisch enthalten waren. In der Kühlfalle waren 3,5 g eines Phenol/Wasser-Gemisches kondensiert.

**Beispiel 5**

a) Belegung eines pulverförmigen Titandioxids mit Palladium und Mangan:

Zu einer Lösung von 82,8 g Mangan(II)-acetylacetonat (0,33 mol) in 750 ml Ethanol wurden bei Raumtemperatur 274,5 g Titandioxid-Pulver (Fa. Norton) gegeben. Dann wurde mit verdünnter Natriumphenolat-Lösung alkalisch gestellt. Die resultierende Suspension wurde abgesaugt und gewaschen. Der mit Mangan dotierte Träger wurde in 1500 ml Wasser aufgeschlämmt und mit 600 ml wäßriger Lösung, enthaltend 50 g Natriumtetrachloropalladat(II)-Lsg. mit 15 % Pd, versetzt. Dann wurde mit verdünnter Natriumphenolat-Lösung alkalisch gestellt. Die nunmehr erhaltene Suspension wurde abgesaugt, gewaschen und bei 100°C getrocknet. Der Kontakt enthielt 2,5 Gew.-% Pd und 6 Gew.-% Mn, jeweils als Metall gerechnet.

b) Einsatz des Träger-Katalysators zur Herstellung von Diphenylcarbonat:

Der Einsatz des Träger-Katalysators zur Herstellung von Diphenylcarbonat erfolgte wie in Beispiel 1. Die Analysen ergaben, daß nach einer Stunde 8,6 % Diphenylcarbonat, nach 2 Stunden 15,2 % Diphenylcarbonat und nach 3 Stunden 18,2 % Diphenylcarbonat im Reaktionsgemisch enthalten waren. In der Kühlfalle waren 15,0 g eines Phenol/Wasser-Gemisches kondensiert.

## Beispiel 6

a) Belegung eines Titandioxid-Extrudats mit Palladium, Kupfer und. Molybdän:

200 ml Titandioxid-Extrudat wurden mit 100 ml 25%iger wäßriger Ammoniak-Lösung vorgetränkt. Anschließend wurde der Träger mit einer Lösung, bestehend aus 300 ml 25%iger wäßriger Ammoniak-Lösung, 1,44 g Palladium(II)-chlorid (0,008 mol), 2,76 g Kupfer(II)-chlorid-2-hydrat (0,016 mol) und 3,04 g Ammoniummolybdat(VI)-4-hydrat (0,0025 mol) versetzt. Die Mischung wurde 1h bei 80°C gerollt und die flüchtigen Bestandteile anschließend bei 80°C im Vakuum abgezogen. Nach Trocknung unter Stickstoff bei 200°C erhielt man einen Katalysator mit 4,3 g Pd, 5,2 g Cu und 8,3 g Mo pro Liter Kontaktmasse.

b) Einsatz des Träger-Katalysators zur Herstellung von Diphenylcarbonat:

Der Einsatz des Träger-Katalysators zur Herstellung von Diphenylcarbonat erfolgte wie in Beispiel 3. Die Analysen ergaben, daß nach einer Stunde 2,2 % Diphenylcarbonat, nach 2 Stunden 4,5 % Diphenylcarbonat und nach 3 Stunden 6,3 % Diphenylcarbonat im Reaktionsgemisch enthalten waren. In der Kühlfalle waren 5,7 g eines Phenol/Wasser-Gemisches kondensiert.

## Beispiel 7

a) Belegung eines pulverförmigen Lanthanoxids mit Palladium und Vanadium

Zu einer HNO$_3$-sauren Lösung aus 13,8 g Ammoniumvanadat (0,12 mol) in 1380 ml H$_2$O wurden bei 70°C 189 g Lanthan(III)-oxid-Pulver (Bayer) gegeben. Dann wurde die Suspension abgesaugt, getrocknet und 4 h bei 400°C getempert. Der mit Vanadium dotierte Träger wurde bei Raumtemperatur zu einer Lösung aus 12,5 g Palladium(II)-bromid (0,05 mol) und 19 g Natriumbromid (0,18 mol) in 1000 ml H$_2$O gegeben. Die Suspension wurde gerührt, abgesaugt, gewaschen und bei 60°C getrocknet.

b) Einsatz des Träger-Katalysators zur Herstellung von Diphenylcarbonat

Der Einsatz des Träger-Katalysators zur Herstellung von Diphenylcarbonat erfolgte wie in Beispiel 1. Die Analysen ergaben, daß nach einer Stunde 2,0 % Diphenylcarbonat, nach 2 Stunden 3,6 % Diphenylcarbonat und nach 3 Stunden 4,7 % Diphenylcarbonat im Reaktionsgemisch enthalten waren. In der Kühlfalle waren 3,5 g eines Phenol/Wasser-Gemisches kondensiert.

## Beispiel 8

a) Belegung eines pulverförmigen Eisenoxids mit Palladium und Mangan

Zu einer Aufschlämmung von 189 g Eisen(III)-oxid (Bayer) in 1000 ml H$_2$O wurden bei Raumtemperatur 200 ml Lösung von 21,6 g Mangan(II)-chlorid-4-hydrat (0,11 mol) in H$_2$O gegeben. Dann wurde mit verdünnter Natronlauge alkalisch gestellt. Die Suspension wurde mit 300 ml Lösung von 12,5 g Palladium(II)-bromid (0,05 mol) und 19 g Natriumbromid (0,18 mol) in H$_2$O versetzt. Dann wurde mit verdünnter Natronlauge alkalisch gestellt. Die Suspension wurde abgesaugt, gewaschen und bei 100°C getrocknet.

b) Einsatz des Träger-Katalysators zur Herstellung von Diphenylcarbonat

Der Einsatz des Träger Katalysators zur Herstellung von Diphenylcarbonat erfolgte wie in Beispiel 1. Die Analysen ergaben, daß nach einer Stunde 2,9 % Diphenylcarbonat, nach 2 Stunden 5,1 % Diphenylcarbonat und nach 3 Stunden 6,7 % Diphenylcarbonat im Reaktionsgemisch enthalten waren. In der Kühlfalle waren 5,1 g eines Phenol/Wasser-Gemisches kondensensiert.

## Beispiel 9

a) Belegung eines pulverförmigen Magnesiumoxids mit Palladium und Mangan

Zu einer Aufschlämmung von 189 g Magnesium(II)-oxid (Bayer) in 1000 ml H$_2$O wurden bei Raumtemperatur 200 ml Lösung von 21,6 g Mangan(II)-chlorid-4-hydrat (0,11 mol) in H$_2$O und 300 ml Lösung von 12,5 g Palladium(II)-bromid (0,05 mol) und 19 g Natriumbromid (0,18 mol) in H$_2$O ge-

geben. Die Suspension wurde gerührt, gewaschen und bei 100°C getrocknet.

b) Einsatz des Träger-Katalysators zur Herstellung von Diphenylcarbonat

Der Einsatz des Träger Katalysators zur Herstellung von Diphenylcarbonat erfolgte wie in Beispiel 1. Die Analysen ergaben, daß nach einer Stunde 1,5 % Diphenylcarbonat, nach 2 Stunden 2,6 % Diphenylcarbonat und nach 3 Stunden 3,3 % Diphenylcarbonat im Reaktionsgemisch enthalten waren. In der Kühlfalle waren 2,5 g eines Phenol/Wasser-Gemisches kondensiert.

## Beispiel 10

a) Belegung einer pulverförmigen Aktivkohle mit Palladium und Mangan

Zu einer Aufschlämmung von 189 g Aktivkohle (Norit) in 1000 ml $H_2O$ wurden bei Raumtemperatur 200 ml Lösung von 21,6 g Mangan(II)-chlorid-4-hydrat (0,11 mol) in $H_2O$ und 300 ml Lösung von 12,5 g Palladium(II)-bromid (0,05 mol) und 19 g Natriumbromid (0,18 mol) in $H_2O$ gegeben. Die Suspension wurde gerührt, abgesaugt, gewaschen und bei 100°C getrocknet.

b) Einsatz des Träger-Katalysators zur Herstellung von Diphenylcarbonat

Der Einsatz des Träger-Katalysators zur Herstellung von Diphenylcarbonat erfolgte wie in Beispiel 1. Die Analysen ergaben, daß nach einer Stunde 5,2 % Diphenylcarbonat, nach 2 Stunden 9,2 % Diphenylcarbonat und nach 3 Stunden 11,9 % Diphenylcarbonat im Reaktionsgemisch enthalten waren. In der Kühlfalle waren 9,0 g eines Phenol/Wasser-Gemisches kondensiert.

## Beispiel 11

a) Belegung eines pulverförmigen Siliciumoxids mit Palladium und Mangan

Zu einer Lösung von 27,7 g Mangan(II)-acetylacetonat (0,11 mol) und 33,3 g Natriumtetrachloropalladat(II)-Lösung mit 15 % Pd in Ethanol wurden 189 g Siliciumdioxid (Tolsa) gegeben. Dann wurde mit verdünnter Natronlauge alkalisch gestellt. Die Suspension wurde gerührt, abgesaugt, gewaschen und bei 100°C getrocknet.

b) Einsatz des Träger-Katalysators zur Herstellung von Diphenylcarbonat

Der Einsatz des Träger-Katalysators zur Herstellung von Diphenylcarbonat erfolgte wie in Beispiel 1. Die Analysen ergaben, daß nach einer Stunde 8,1 %. Diphenylcarbonat, nach 2 Stunden 14,3 % Diphenylcarbonat und nach 3 Stunden 18,6 % Diphenylcarbonat im Reaktionsgemisch enthalten waren. In der Kühlfalle waren 14,1 g eines Phenol/Wasser-Gemisches kondensiert.

## Beispiel 12

a) Belegung eines pulverförmigen Aluminiumoxids mit Palladium und Mangan

Zu einer Lösung von 27,7 g Mangan(II)-acetylacetonat (0,11 mol) und 33,3 g Natriumtetrachloropalladat(II)-Lösung mit 15 % Pd in Ethanol wurden 189 g Aluminium(III)-oxid (Rhone Poulenc) gegeben. Dann wurde mit verdünnter Natronlauge alkalisch gestellt. Die Suspension wurde gerührt, abgesaugt, gewaschen und bei 100°C getrocknet.

b) Einsatz des Träger-Katalysators zur Herstellung von Diphenylcarbonat

Der Einsatz des Träger-Katalysators zur Herstellung von Diphenylcarbonat erfolgte wie in Beispiel 1. Die Analysen ergaben, daß nach einer Stunde 5,8 % Diphenylcarbonat, nach 2 Stunden 10,2 % Diphenylcarbonat und nach 3 Stunden 13,2 % Diphenylcarbonat im Reaktionsgemisch enthalten waren. In der Kühlfalle waren 10,0 g eines Phenol/Wasser-Gemisches kondensiert.

## Beispiel 13

a) Belegung eines pulverförmigen Manganoxids mit Palladium und Mangan

Zu einer Lösung von 27,7 g Mangan(II)-acetylacetonat (0,11 mol) und 10,4 g Palladium(II)-acetat (0,05 mol) in Ethanol wurden 189 g Mangan(IV)-oxid (Fluka) gegeben. Die Suspension wurde im Rotationsverdampfer eingedampft.

b) Einsatz des Träger-Katalysators zur Herstellung von Diphenylcarbonat

Der Einsatz des Träger-Katalysators zur Herstellung von Diphenylcarbonat erfolgte wie in Beispiel 1. Die Analysen ergaben, daß nach einer Stunde 3,7 % Diphenylcarbonat, nach 2 Stunden 6,6 % Diphenylcarbonat und nach 3 Stunden 8,6 % Diphenylcarbonat im Reaktionsgemisch enthalten waren. In der Kühlfalle waren 6,5 g eines Phenol/Wasser-Gemisches kondensiert.

**Beispiel 14**

a) Belegung eines pulverförmigen Manganoxids mit Palladium und Kobalt

Zu einer Lösung von 12,5 g Palladium(II)-bromid (0,05 mol), 24,2 g Kobalt(II)-chlorid-6-hydrat (0,1 mol) und 19 g Natriumbromid (0,18 mol) in $H_2O$ wurden 189 g Mangan(IV)-oxid (Fluka) gegeben. Die Suspension wurde gerührt, mit Natronlauge alkalisch gestellt, abgesaugt, gewaschen und bei 100°C getrocknet.

b) Einsatz des Träger-Katalysators zur Herstellung von Diphenylcarbonat

Der Einsatz des Träger-Katalysators zur Herstellung von Diphenylcarbonat erfolgte wie in Beispiel 1. Die Analysen ergaben, daß nach einer Stunde 3,5 % Diphenylcarbonat, nach 2 Stunden 6,1 % Diphenylcarbonat und nach 3 Stunden 8,0 % Diphenylcarbonat im Reaktionsgemisch enthalten waren. In der Kühlfalle waren 6,1 g eines Phenol/Wasser-Gemisches kondensiert.

**Beispiel 15**

a) Belegung von Aktivkohle-Extrudaten mit Palladium und Mangan

200 ml Aktivkohle-Extrudate (Norit) wurden mit 58 ml Tränkflüssigkeit, enthaltend 21,6 g Mangan (II)-chlorid-4-hydrat (0,11 mol) und 33,3 g Natriumtetrachloropalladat(II)-Lösung mit 15 % Pd in $H_2O$, getränkt. Dann wurde unter Stickstoff getrocknet.

b) Einsatz des Träger-Katalysators zur Herstellung von Diphenylcarbonat

Der Einsatz des Träger-Katalysators zur Herstellung von Diphenylcarbonat erfolgte wie in Beispiel 1, jedoch mit dem Unterschied, daß sich der Katalysator ortsfest in einem Maschendrahtkörbchen befand. Die Analysen ergaben, daß nach einer Stunde 3,2 % Diphenylcarbonat, nach 2 Stunden 5,6 % Diphenylcarbonat und nach 3 Stunden 7,3 % Diphenylcarbonat im Reaktionsgemisch enthalten waren. In der Kühlfalle waren 5,5 g eines Phenol/Wasser-Gemisches kondensiert.

**Beíspiel 16**

a) Belegung eines pulverförmigen Montmorillonites mit Palladium und Mangan

Zu einer Lösung von 27,7 g Mangan(II)-acetylacetonat (0,11 mol) in Ethanol wurden 189 g Montmorillonit (Fluka) gegeben. Dann wurde mit verdünnter Natronlauge alkalisch gestellt. Die Suspension wurde abgesaugt und gewaschen. Der mit Mangan dotierte Träger wurde in 1000 ml $H_2O$ aufgeschlämmt und mit 33,3 g Natriumtetrachloropalladat(II)-Lösung mit 15 % Pd versetzt. Die Suspension wurde abgesaugt und bei 100°C getrocknet.

b) Einsatz des Träger-Katalysators zur Herstellung von Diphenylcarbonat

Der Einsatz des Träger-Katalysators zur Herstellung von Diphenylcarbonat erfolgte wie in Beispiel 1. Die Analysen ergaben, daß nach einer Stunde 3,4 % Diphenylcarbonat, nach 2 Stunden 6,0 % Diphenylcarbonat und nach 3 Stunden 7,8 % Diphenylcarbonat im Reaktionsgemisch enthalten waren. In der Kühlfalle waren 5,9 g eines Phenol/Wasser-Gemisches kondensiert.

**Patentansprüche**

1. Verwendung eines Katalysators enthaltend

(i) ein Platinmetall, ein Platinmetall-Halogenid oder eine Platinmetall-Halogenid enthaltende Komplexverbindung oder eine Verbindung, die unter Reaktionsbedingungen in ein Platinmetall, ein Platinmetall-Halogenid oder eine Platinmetall-Halogenid enthaltende Komplexverbindung übergeführt werden kann, in einer Menge von 0,01 bis 15 Gew.-%, gerechnet als Platinmetall und bezogen auf das Gesamtgewicht des Katalysators,

(ii) eine cokatalytisch wirkende Metallverbindung eines Elementes der Gruppe Mn, Cu, Co, V, Mo in einer Menge von 0,01 bis 15 Gew.-%, gerechnet als Metall und bezogen auf das Gesamtgewicht des Katalysators;

(iii) Trägermaterial;

(iv) und gegebenenfalls 0,5 bis 99,5 Gew.-% Bindemittel, bezogen auf das Gesamtgewicht des Trägers,

als Katalysator bei der Herstellung eines aromatischen Carbonats der Formel

$$R\text{-}O\text{-}CO\text{-}O\text{-}R,$$

in der

R substituiertes oder nicht substituiertes $C_6\text{-}C_{12}$-Aryl, bevorzugt substituiertes oder nicht substituiertes Phenyl, besonders bevorzugt nicht substituiertes Phenyl bedeutet,

durch Umsetzung einer aromatischen Hydroxyverbindung der Formel

$$R\text{-}OH$$

in der R die obige Bedeutung hat,
mit Kohlenmonoxid und Sauerstoff in Gegenwart eines quartären Ammonium- oder Phosphonium-Salzes und einer Base bei 30 bis 200°C und bei einem Druck von 1 bis 150 bar.

2. Verwendung gemäß Anspruch 1, dadurch gekennzeichnet, dass das Trägermaterial als Katalysatorträger auf Basis von Kohle, Elementoxiden, Elementcarbiden oder Elementsalzen vorliegt.

3. Verwendung nach Anspruch 1, dadurch gekennzeichnet, dass das Bindemittel als $SiO_2$, $Al_2O_3$ oder Tonmineralien vorliegt.

4. Verwendung nach Anspruch 1, dadurch gekennzeichnet, dass als Platinmetall Pd oder Rh, als Metall, Metallhalogenid oder Metallhalogenid enthaltende Komplexverbindung vorliegt.

5. Verwendung nach Anspruch 1, dadurch gekennzeichnet, dass man den Träger-Katalysator in einer kontinuierlichen Fahrweise im Gegen- oder Gleichstrom oder mit ortsfesten Katalysatoren in Rührkessel, Blasensäulenreaktoren oder in der Rieselphase am Festbett-Katalysator mit einer Belastung von 0,01 bis 20 g aromatische Hydroxyverbindung pro Gramm Träger-Katalysator und Stunde, bevorzugt 0,05 bis 10 g aromatische Hydroxyverbindung pro Gramm Träger-Katalysator und Stunde, besonders bevorzugt 0,1 bis 5 g aromatische Hydroxyverbindung pro Gramm Träger-Katalysator und Stunde beaufschlagt, und beim Arbeiten als Suspension in Rührgefäßen oder Blasensäulen in Mengen von 0,001 bis 50 Gew.-%, bevorzugt von 0,01 bis 20 Gew.-%, besonders bevorzugt von 0,1 bis 10 Gew.-%, bezogen auf die eingesetzte Menge an aromatischer Hydroxyverbindung, verwendet.

6. Verwendung nach Anspruch 1, dadurch gekennzeichnet, dass man als Base ein tertiäres Amin, Alkaliphenolat oder Alkalisalz schwacher Säuren, vorzugsweise Alkalicarboxylate und/oder -phenolate, besonders bevorzugt Natriumphenolat verwendet.

7. Verwendung nach Anspruch 1, dadurch gekennzeichnet, dass man als quartäres Salz Tetraalkylammonium- oder -phosphoniumsalze, bevorzugt Tetraalkylammoniumsalze, besonders bevorzugt Tetrabutylammoniumbromid einsetzt.

## Claims

1. The use of a catalyst containing

   (i) a platinum metal, a platinum metal halide or a complex compound which contains a platinum metal halide, or a compound which under reaction conditions can be converted into a platinum metal, a platinum metal halide or a complex compound which contains a platinum metal, in an amount of 0.01 to 15 % by weight, calculated as platinum metal and with respect to the total weight of the catalyst,

   (ii) a metal compound, which acts as a co-catalyst, of an element of the group comprising Mn, Cu, Co, V, Mo in an amount of 0.01 to 15 % by weight, calculated as metal and with respect to the total weight of the catalyst;

   (iii) a support material;

   (iv) and optionally 0.5 to 99.5 % by weight of a binder with respect to the total weight of the support,

   as a catalyst for the production of an aromatic carbonate of formula

$$R\text{-}O\text{-}CO\text{-}O\text{-}R,$$

   wherein

   R denotes a substituted or unsubstituted $C_6\text{-}C_{12}$ aryl, preferably a substituted or unsubstituted phenyl, most preferably an unsubstituted phenyl,

   by the reaction of an aromatic hydroxy compound of formula

$$R\text{-}OH,$$

   wherein R has the above meaning,
   with carbon monoxide and oxygen in the presence of a quaternary ammonium or phosphonium salt and a base at 30 to 200°C and at a pressure of 1 to 150 bar.

2. A use according to claim 1, characterised in that the support material is a catalyst support based on carbon, oxides of elements, carbides of elements or salts of elements.

**3.** A use according to claim 1, characterised in that the binder is SiO$_2$, Al$_2$O$_3$ or clay minerals.

**4.** A use according to claim 1, characterised in that Pd or Rh is the platinum metal, and exists as a metal, as a metal halide or as a complex compound which contains a metal halide.

**5.** A use according to claim 1, characterised in that the supported catalyst is subjected, in a continuous mode of operation in counter-current or co-current flow, or using fixed catalysts in a stirred vessel, in bubble column reactors or in a percolating phase through a fixed bed catalyst, to a loading of 0.01 to 20 g per hour of aromatic hydroxy compound per gram of supported catalyst, preferably 0.05 to 10 g per hour of aromatic hydroxy compound per gram of supported catalyst, most preferably 0.1 to 5 g per hour of aromatic hydroxy compound per gram of supported catalyst, and when employed as a suspension in stirred vessels or bubble columns it is used in amounts of 0.001 to 50 % by weight, preferably 0.01 to 20 % by weight, most preferably 0.1 to 10 % by weight, with respect to the amount of aromatic hydroxy compound used.

**6.** A use according to claim 1, characterised in that a tertiary amine, alkali phenolate or alkali salts of weak acids, preferably alkali carboxylates and/or phenolates, most preferably sodium phenolate, are used as a base.

**7.** A use according to claim 1, characterised in that tetraalkylammonium or phosphonium salts, preferably tetraalkylammonium salts, most preferably tetrabutylammonium bromide, are used as a quaternary salt.

**Revendications**

**1.** Utilisation d'un catalyseur contenant

(i) un métal de la série du platine, un halogénure d'un métal de la série du platine ou un complexe contenant un halogénure d'un métal de la série du platine ou un composé qui, dans les conditions de réaction, peut être converti en un métal de la série du platine, un halogénure d'un métal de la série du platine ou un complexe contenant un halogénure d'un métal de la série du platine, en quantité de 0,01 à 15 % en poids exprimé en métal de la série du platine et rapporté au poids total du catalyseur,
(ii) un dérivé métallique, agissant en tant que catalyseur auxiliaire, d'un élément du groupe consistant en Mn, Cu, Co, V, Mo, en quantité de 0,01 à 15 % en poids, exprimé en métal et rapporté au poids total du catalyseur ;
(iii) une matière de support ;
(iv) et le cas échéant 0,5 à 99,5 % en poids d'un liant, par rapport au poids total du support,

en tant que catalyseur à la préparation d'un carbonate aromatique de formule

$$R-O-CO-O-R,$$

dans laquelle
R représente un groupe aryle substitué ou non en C$_6$-C$_{12}$, de préférence un groupe phényle substitué ou non, dans les meilleures conditions le groupe phényle non substitué,
par réaction d'un composé aromatique hydroxylé de formule

$$R-OH$$

dans laquelle R a les significations indiquées ci-dessus,
avec le monoxyde de carbone et l'oxygène en présence d'un sel d'ammonium ou de phosphonium quaternaire et d'une base à des températures de 30 à 200°C et des pressions de 1 à 150 bar.

**2.** Utilisation selon la revendication 1, caractérisée en ce que la matière de support servant de support du catalyseur est à base de charbon, d'oxydes d'éléments, de carbures d'éléments ou de sels d'éléments.

**3.** Utilisation selon la revendication 1, caractérisée en ce que le liant consiste en SiO$_2$, Al$_2$O$_3$ ou minéral argileux.

**4.** Utilisation selon la revendication 1, caractérisée en ce que le métal de la série du platine est Pd ou Rh, à l'état de métal, d'halogénure du métal ou de complexe contenant un halogénure du métal.

**5.** Utilisation selon la revendication 1, caractérisée en ce que l'on utilise le catalyseur sur support dans un mode opératoire continu à contre-courant ou en courants parallèles avec le catalyseur en disposition fixe dans des récipients équipés de dispositifs d'agitation, des réacteurs à colonnes à bulles ou en phase ruisselante sur le catalyseur en lit fixe à une charge de 0,01 à 20 g de composé aromatique hydroxylé par gramme du catalyseur sur support et par heure, de préférence de 0,05 à 10 g de composé aromatique hydroxylé par gramme du catalyseur sur support et par heure, plus spécialement de 0,1 à 5 g de composé aromatique hydroxylé par gramme de catalyseur sur support et par heure, et dans des opérations en suspension dans des récipients équipés de dispositifs d'agitation ou des colonnes à bulles en quantités de 0,001 à 50 % en poids, de préférence de 0,01 à 20 % en poids, plus spécialement de 0,1 à 10 % en poids, par rapport à la quantité de composé aromatique hydroxylé mise en oeuvre.

6. Utilisation selon la revendication 1, caractérisée en ce que la base utilisée est une amine tertiaire, un phénolate alcalin ou un sel alcalin d'acide faible, de préférence un carboxylate et/ou un phénolate alcalin, dans les meilleures conditions le phénolate de sodium.

7. Utilisation selon la revendication 1, caractérisée en ce que le sel quaternaire utilisé est un sel de tétraalkyl-ammonium ou -phosphonium, de préférence un sel de tétraalkylammonium et dans les meilleures conditions le bromure de tétrabutylammonium.